# FASCICULE DE BREVET EUROPEEN

(11) **EP 3 101 335 B1**
(45) Date de publication et mention de la délivrance du brevet: **09.05.2018**
(21) Numéro de dépôt: 16170284.0
(22) Date de dépôt: 19.05.2016
(51) Int. Cl.: F21V 7/04, F21V 7/05, F21V 7/22, F21V 13/04, G02B 27/10, F21W 131/205, F21Y 115/10, F21V 5/04

(54) **DISPOSITIF D'ECLAIRAGE A COUPOLE D'ECLAIRAGE D'ENCOMBREMENT REDUIT POUR FORMER UNE TACHE D'ECLAIREMENT A DIAMETRE ET TEMPERATURE DE COULEUR VARIABLES**
BELEUCHTUNGSVORRICHTUNG MIT KOMPAKTER LICHTKUPPEL ZUR BILDUNG EINES LICHTFLECKS MIT VARIABLE DURCHMESSERS UND FARBTEMPERATUR
A LIGHTING DEVICE HAVING A COMPACT LIGHTING DOME FOR FORMING AN ILLUMINATION SPOT OF VARIABLE DIAMETER AND OF VARIABLE COLOR TEMPERATURE

(30) Priorité: 02.06.2015 FR 1554975
(43) Date de publication de la demande: 07.12.2016
(73) Titulaire: Maquet SAS, 45160 Ardon (FR)
(72) Inventeur: VU THI, Minh Hong, 45160 ARDON (FR); VALTEAU, Cécilia, 45160 ARDON (FR)
(74) Mandataire: Prugneau, Philippe

(56) Documents cités:
- EP-A1- 2 799 764
- WO-A1-2014/087088
- FR-A- 1 025 846
- FR-A1- 2 194 915
- FR-A2- 2 339 129
- US-A- 5 913 599

## Description

### Domaine technique

Le domaine de l'invention concerne les dispositifs d'éclairage, en particulier les dispositifs d'éclairage à coupole d'éclairage axial pour éclairer un champ opératoire médical.

### Technique antérieure

En milieu médical, notamment en bloc opératoire, les conditions d'éclairage doivent être adaptées pour qu'un utilisateur, par exemple un chirurgien ou un médecin puisse travailler correctement. En particulier, un bon éclairage doit se conformer à certaines normes et fournir une lumière présentant un indice de rendu des couleurs (IRC ou Ra) compris entre 85 et 100 ainsi qu'une température de couleur comprise entre 3000 Kelvin (K) et 6700K. Plus précisément, on désigne ici par « température de couleur » d'une lumière, la température de couleur équivalente évaluée de manière classique à partir des coordonnées chromatiques (x,y) du spectre de la lumière dans un diagramme chromatique de la Commission Internationale de l'Eclairage.

En outre, en milieu médical, un bon éclairage doit offrir un éclairement homogène, un rendement optique élevé, sans créer d'ombres colorées dans le champ d'éclairage. Un bon éclairage doit aussi permettre au chirurgien de faire varier la température de couleur et/ou la dimension de la tache d'éclairement pour l'adapter à son besoin.

De plus, dans le bloc opératoire, le dispositif d'éclairage comporte généralement une embase fixée au plafond et de laquelle s'étend un bras articulé portant une coupole d'éclairage dans laquelle sont disposés une pluralité de modules d'éclairage. Ainsi, un minimum d'encombrement de la coupole d'éclairage au dessus du champ opératoire est recherché pour permettre une bonne maniabilité de la coupole d'éclairage par l'utilisateur.

On connait du document EP 2 799 764 du demandeur un dispositif d'éclairage pour former une tache d'éclairement à température de couleur et diamètre variables. Ce dispositif permet en outre grâce à un diviseur de faisceau de mélanger des faisceaux de lumière blanche de température de couleur différente issus de deux sources de lumière blanche pour générer des faisceaux de lumière mélangée résultants à une température de couleur intermédiaire. Le système optique de ce dispositif d'éclairage comprend plusieurs éléments optiques pour focaliser et combiner les faisceaux de lumière mélangée et former une tache d'éclairement à une température de couleur intermédiaire sur le champ d'éclairage. Ces éléments optiques sont un réflecteur elliptique et une lentille, qui peuvent être montés mobiles par rapport au diviseur de faisceaux. De plus, dans ce dispositif d'éclairage afin de diminuer la divergence des faisceaux de lumière blanche issus des sources de lumière, des lentilles sont installées entre les sources de lumière et le diviseur de faisceaux. Ce dispositif d'éclairage présente comme inconvénients de comprendre d'une part une multitude d'éléments optiques massiques à intégrer dans la coupole d'éclairage qui en complexifie et alourdit la structure, et d'autre part de comprendre un réflecteur elliptique, qui est une pièce optique de conception technique complexe et onéreuse. Le document WO 2014 087088 A1 divulgue un dispositif d'éclairage à LEDs notamment pour un appareil d'éclairage médical d'un champ opératoire.

### Exposé de l'invention

Le but de l'invention est donc de remédier à ces inconvénients en proposant un dispositif d'éclairage comprenant une coupole d'éclairage présentant une meilleur maniabilité et un cout réduit, tout en offrant toujours un éclairement homogène, un rendement optique élevé, sans créer d'ombres colorées dans le champ d'éclairage et autorisant une variation de la température de couleur de l'éclairage et aussi une variation de la dimension de la tache d'éclairement.

A cet effet, l'invention a pour objet un dispositif d'éclairage pour l'éclairage d'un champ opératoire comprenant dans une coupole à éclairage axial une première couronne de LEDs apte à émettre un premier faisceau de lumière à une première température de couleur et une seconde couronne de LEDs, coaxiale à la première couronne de LEDs et apte à émettre un second faisceau de lumière à une seconde température de couleur différente de la première température de couleur et un miroir annulaire à facettes semi-réfléchissantes coaxial pour mélanger les faisceaux de lumière émis par les deux couronnes de LEDs et former deux faisceaux de lumière mélangée résultants ayant une même température de couleur intermédiaire comprise entre la première température de couleur et la seconde température de couleur, et un système optique annulaire qui entoure le miroir annulaire à facettes semi-réfléchissantes pour réfléchir le premier faisceau de lumière mélangée résultant dans le champ opératoire pour le combiner avec le second faisceau de lumière mélangée résultant suivant une certaine configuration de superposition dans un plan de superposition où est formée une tache d'éclairement à la température de couleur intermédiaire (Tkr), caractérisé en ce que le système optique annulaire qui entoure le miroir annulaire à facettes semi-réfléchissantes est un miroir annulaire à réflexion totale à facettes planes sensiblement homothétiques respectivement aux facettes semi-réfléchissantes et en ce qu'il est prévu des optiques de collimation entre chaque couronne de LED et le miroir annulaire à facettes semi-réfléchissantes.

Avec cet agencement, les faisceaux de lumière issus des LEDs et atteignant le miroir annulaire à facettes semi-réfléchissantes sont très intensifs car ils sont collimatés dès leur sortie des LEDs. Ainsi, par rapport à l'art antérieur, la coupole d'éclairage de la présente invention ne présente plus de lentille massique entre les sources de lumière et le miroir annulaire à facettes semi-réfléchissantes. De plus, le miroir annulaire à réflexion totale ne participe plus à la construction du faisceau hautement intensif mais ici sert uniquement à le dévier. Un miroir annulaire à facettes planes est peu onéreux à fabriquer. Le nombre de pièces optiques dans la coupole d'éclairage axial de la présente invention est réduit par rapport à l'art antérieur. En effet, la coupole d'éclairage ne nécessite plus de lentille en aval du miroir annulaire à facettes semi-réfléchissantes pour combiner sur le champ opératoire le second faisceau de lumière mélangée résultant avec le premier faisceau de lumière mélangée résultant.

Le dispositif d'éclairage selon l'invention peut ainsi avantageusement présenter les particularités suivantes :
- les optiques de collimation sont des lentilles ;
- les optiques de collimation sont des collimateurs ;
- les optiques de collimation sont des réflecteurs hybride ;
- une alimentation électrique est prévue pour alimenter en courants les LEDs et moduler lesdits courants ;
- dans la première couronne de LEDs, les LEDs sont couplées de manière alternée à une première et un seconde optique de collimation, la seconde optique de collimation étant différente de la première optique de collimation et dans la seconde couronne de LEDs, les LEDs sont couplées de manière alternée à une troisième et une quatrième optique de collimation, les première et troisième optiques de collimation étant identiques et les seconde et quatrième optiques de collimation étant identiques, pour former respectivement une première tache d'éclairement et une seconde tache d'éclairement de diamètres différents ; avec cet agencement, on peut moduler le diamètre de la tache d'éclairement sans mouvement mécanique dans le dispositif d'éclairage et en particulier avec des sources lumineuses qui sont statiques par rapport au diviseur de faisceau ce qui simplifie la construction du dispositif d'éclairage tout en gardant l'éclairement constant par un pilotage de l'intensité des courants d'alimentation dans les sources lumineuses ;
- l'alimentation électrique pour alimenter en courants les LEDs et moduler les courants permet de modifier la dimension de la tache d'éclairement et/ou de modifier la température de couleur de la tache d'éclairement ;
- l'alimentation électrique est agencée en outre pour moduler les courants de telle façon à former une tache d'éclairement à un niveau d'éclairement constant.

Avec cet agencement l'épaisseur, la complexité de la structure et le poids de la coupole sont réduits, tout en gardant la possibilité de faire varier la température de la tache d'éclairement et de modifier le diamètre de la tache d'éclairement. La variation de la température de couleur de la tache d'éclairement est obtenue en pilotant l'intensité des courants d'alimentation dans les sources de lumière à LEDs.

### Description sommaire des dessins

La présente invention sera mieux comprise et d'autres avantages apparaîtront à la lecture de la description détaillée de plusieurs modes de réalisation pris à titre d'exemples nullement limitatifs et illustrés par les dessins annexés dans lesquels :
- la figure 1 est une vue en perspective d'un dispositif d'éclairage selon l'invention utilisé dans un bloc opératoire ;
- la figure 2 est une représentation schématique du montage du diviseur de faisceau ;
- la figure 3 est une vue en perspective schématique des éléments optiques à monter dans une coupole d'éclairage selon l'art antérieur ;
- la figure 4 est une vue en perspective schématique des éléments optiques agencés dans une coupole d'éclairage selon l'art antérieur ;
- la figure 5 est une vue en perspective schématique des éléments optiques à monter dans une coupole d'éclairage selon un mode de réalisation de l'invention ;
- la figure 6 est une vue en perspective schématique des éléments optiques agencés dans une coupole d'éclairage selon un mode de réalisation de l'invention ;
- la figure 7 est une représentation schématique du système optique et des sources de lumière pour former une tache d'éclairement sur le champ opératoire selon un mode de réalisation de l'invention;
- la figure 8 est une représentation schématique des sources de lumière et du système optique selon un autre mode de réalisation du dispositif d'éclairage selon l'invention.

### Description d'un exemple de réalisation

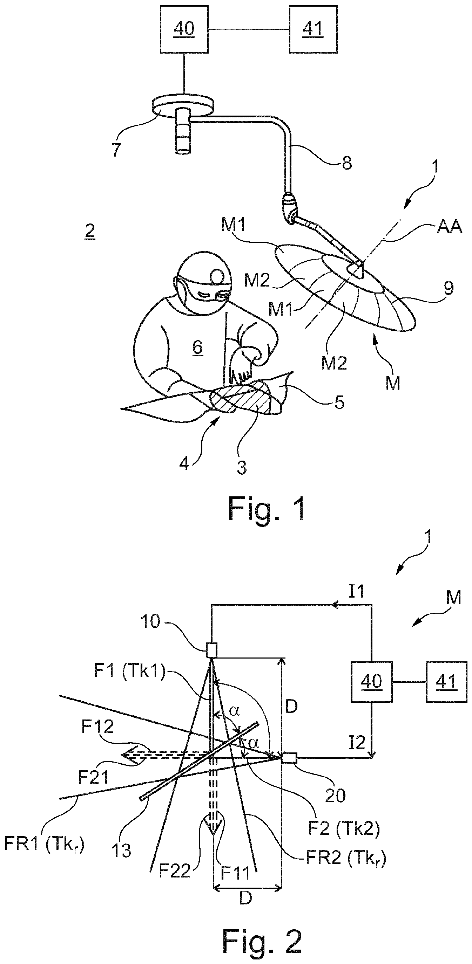

En référence à la Figure 1, le dispositif d'éclairage 1 selon l'invention est en particulier destiné à être utilisé dans un bloc opératoire 2 pour former une tache d'éclairement 3 (schématisée par des hachures) dans un champ opératoire 4, par exemple sur le corps d'un patient 5 opéré par un chirurgien 6. Dans cet exemple et de manière connue, le dispositif d'éclairage 1 comporte une embase 7 fixée au plafond du bloc opératoire 2 et de laquelle s'étend un bras articulé 8 portant une coupole d'éclairage 9 dans laquelle sont disposés par exemple une pluralité de modules d'éclairage M qui peuvent être différents, pourvus ici de LEDs (non visibles sur cette figure) qui se présentent chacun ici sous la forme d'un quartier de la coupole pour fournir une tache d'éclairement 3 centrée dans l'axe d'éclairement AA de la coupole. Le champ opératoire 4 peut être situé entre 0.8m et 1.6m de la coupole d'éclairage 9. La tache d'éclairement 3 peut avoir un diamètre de 5 à 30 centimètres.

Le dispositif d'éclairage 1 selon l'invention est prévu pour former une tache d'éclairement 3 à dimension et à température de couleur variables à partir des modules de lumière M, chaque module de lumière comportant deux sources de lumière ayant des température de couleur différentes, par exemple deux LEDs blanches (ou diode électroluminescente) disposées symétriquement par rapport à un diviseur de faisceau. Plus particulièrement, cette tache d'éclairement est formée par la superposition des éclairages centrés sur l'axe AA produits par les différents modules de lumière M.

Les sources de lumière dans les différents modules M1, M2 sont ici alimentées sélectivement et individuellement en courant par une unité d'alimentation électrique 40 couplée à une unité de contrôle/commande 41.

Il est connu de l'art antérieur le principe de la division des faisceaux lumineux produits par deux LEDs dans un module de lumière M. Comme cela est rappelé dans la Figure 2, la première LED 10 est apte à émettre un premier faisceau F1 de lumière (schématisé par un double trait continu) ayant une première température de couleur Tk1. La seconde LED 20 est apte à émettre un second faisceau F2 de lumière (schématisé par un trait simple continu) ayant une seconde température de couleur Tk2 différente de la première température de couleur Tk1. Comme illustré sur la Figure 2, les première et seconde LEDs 10, 20 sont orientées de sorte que les axes médians des faisceaux F1, F2 soient orientés à 90° l'un par rapport à l'autre et on comprend donc que ces LEDs sont disposées symétriquement par rapport au diviseur de faisceau 13.

Le diviseur de faisceau 13 est par exemple un miroir dichroïque ou semi-réfléchissant à haute efficacité, neutre sur le plan spectral et comprenant une lame support (en verre ou en matière synthétique) recouverte de plusieurs couches minces (par traitements optiques pouvant être réalisés dans des bâtis sous vide ou par voie sol gel). Le diviseur de faisceau 13 est apte à diviser chacun des premier et second faisceau F1, F2 en une première partie de faisceau F11, F21 (schématisées respectivement par un double trait continu et par un trait simple continu) transmis par le diviseur de faisceau 13, et en une seconde partie de faisceau F12, F22 (schématisées respectivement par un double trait pointillé et par un trait simple pointillé) réfléchis par le diviseur de faisceau 13. Le diviseur de faisceau 13 doit permettre de diviser chaque faisceau F1, F2 avec un rendement théorique de 100%, c'est-à-dire sans perte, dont par exemple 50% en réflexion et 50% en transmission ou encore par exemple 30% en réflexion et 70% en transmission.

Généralement, le diviseur de faisceau 13 est disposé à égale distance D des LEDs 10, 20 et forme un même angle α de 45° avec chacun des faisceaux F1, F2. De plus, le diviseur de faisceau 13 et les LEDs 10, 20 sont agencés spatialement de sorte que le premier faisceau F1 et le second faisceau F2 atteignent le diviseur de faisceau 13, à l'opposé l'un de l'autre, de part et d'autre du diviseur de faisceau 13. Ainsi, la seconde partie F12 du premier faisceau F1 se superpose ou se combine à ladite première partie F21 du second faisceau F2 pour former un premier faisceau résultant FR1 ayant une température de couleur intermédiaire résultante Tkr comprise entre les première et seconde températures de couleur Tk1, Tk2. De plus, la première partie F11 du premier faisceau F1 se superpose ou se combine à la seconde partie F22 du second faisceau F2 pour former un second faisceau résultant FR2 ayant une même température de couleur résultante Tkr.

Pour faire varier la température de couleur, les LEDs 10,20 sont reliées électriquement à une alimentation électrique 40 couplée à une unité de contrôle/commande 41 (UC) apte à piloter l'alimentation électrique 40 pour alimenter la première LED 10 avec un premier courant électrique I1 et alimenter la seconde LED 20 avec un second courant électrique I2 qui peut être différent de I1.

L'alimentation électrique 40 peut se présenter sous la forme d'une alimentation électrique unique pour toutes les LEDs des modules M ou sous la forme de deux alimentations électriques distinctes alimentant sélectivement respectivement toutes les LEDs 10 et toutes les LEDs 20. Il est connu que le flux lumineux d'une LED dépend de l'intensité du courant qui la traverse. Pour réaliser la modulation de température de couleur, l'alimentation électrique 40 est pilotée par l'UC 41 de sorte à moduler l'intensité des premier et second courants électriques I1, I2 selon le principe des vases communicants.

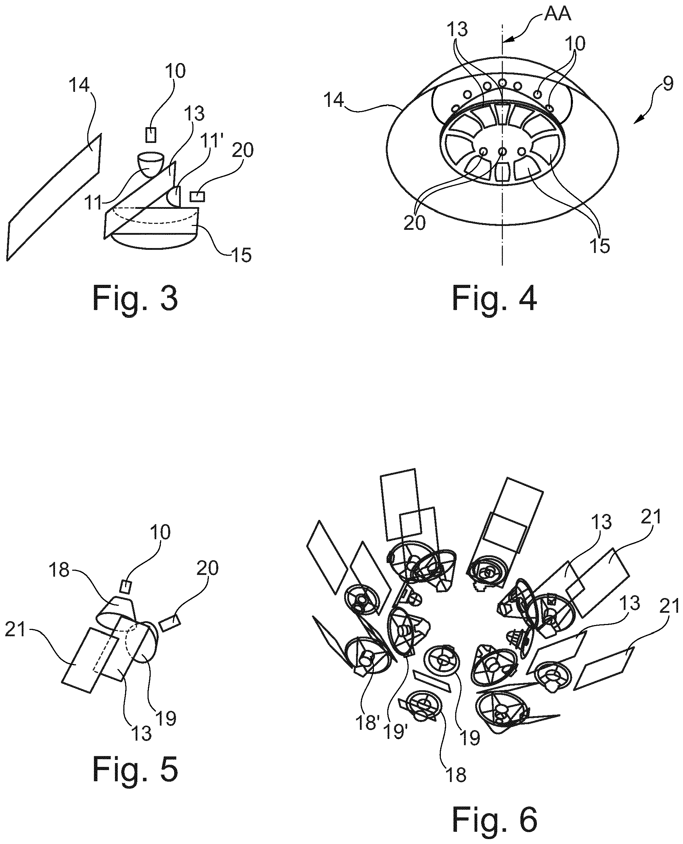

Sur la Figure 3 sont montrés les LEDs 10,20, le diviseur de faisceau 13 ainsi que les moyens optiques d'un module de lumière pour former la tache d'éclairement dans le plan de travail selon l'art antérieur. Chaque module de lumière comprend un réflecteur elliptique 14, une lentille 15 et deux lentilles 11, 11', lesquels sont agencés dans la coupole d'éclairage de sorte à focaliser ensemble les faisceaux FR1 et FR2 et les combiner dans un plan de superposition pour former la tache d'éclairement. Les lentilles 11, 11' sont positionnées entre chaque LED et le diviseur de faisceau pour collecter un maximum de flux lumineux issu de la LED et aussi diminuer la divergence du faisceau. Avec l'utilisation des lentilles 11, 11' l'image virtuelle des LEDs 10, 20 est confondue avec le foyer l'objet du réflecteur elliptique 14 et de la lentille 15.

On a illustré sur la Figure 4 une coupole à éclairage axial selon l'art antérieur dans laquelle plusieurs modules de lumière sont répartis autour de l'axe d'éclairement. Les LEDs 10 des modules de lumière M forment une première couronne autour de l'axe d'éclairement AA et les LEDs 20 de ces modules de lumière M forment une seconde couronne autour de l'axe AA coaxiale à la première couronne de LEDs 10. On a aussi dans la coupole 9 une série de diviseur de faisceau 13 répartis autour de l'axe d'éclairement AA et qui forment une couronne de diviseurs de faisceau coaxiale aux couronnes de LEDs 10,20 comme visible sur la figure 4.

On sait qu'avec une coupole à éclairage axial de ce type on peut former une tache d'éclairement 3 avec une température de couleur variable Tkr comprise entre Tk1 et Tk2 en pilotant l'intensité des courants d'alimentation dans les LEDs 10,20.

On sait aussi qu'avec une coupole à éclairage axial de l'art antérieur du type décrit ci-dessus on peut faire varier le diamètre de la tache d'éclairement en faisant varier l'agencement des LEDs et/ou du système optique par rapport au diviseur de faisceau, ou en pilotant l'intensité des courants d'alimentation dans les LEDs.

Pour avoir une haute efficacité, le réflecteur elliptique est de fabrication complexe et couteuse, car il est généralement préparé en utilisant la technologie de l'injection plastique. La préparation du moule d'injection est un investissement couteux. La pièce plastique produite doit être ensuite traitée sous vide pour y déposer successivement une couche d'accroche puis plusieurs couches métalliques. Le réflecteur elliptique étant une pièce non plane, cela implique une complexité pour le dépôt uniforme des couches métalliques.

Le réflecteur elliptique peut aussi être fabriqué en aluminium avec la technique de repoussage, cette technologie étant onéreuse et connue pour avoir un défaut de forme moins précis que l'injection plastique. De plus, comme le réflecteur elliptique n'est pas à symétrie de révolution, il faut partir d'une pièce à symétrie de révolution que l'on doit découper ce qui implique des opérations industrielles complexes et pouvant risquer des détériorations. Enfin la réflexion n'est que de 80 %

Il est aussi à noter que dans la coupole à éclairage axial de l'art antérieur, pour donner une tache de lumière plus grande, l'image virtuelle de la LED est déplacée hors de l'image objet du réflecteur elliptique 14 et de la lentille 15. Ce principe engendre une tache de lumière non ronde venant du côté du réflecteur elliptique qui fait que la superposition de cette tache avec celle venant de la lentille 15 donne une tache de lumière finale non homogène.

Malgré la présence des lentilles 11, 11' positionnées entre chaque LED et le diviseur de faisceau 13 l'épaisseur de la coupole à éclairage axial reste importante par l'utilisation d'une combinaison des optiques lentille 11 et réflecteur elliptique 14 ou lentille 11' et lentille 15 comme optiques de focalisation. La taille des optiques 14 et 15 est très importante pour récupérer le maximum des faisceaux de lumière résultants. Le poids et volume de la coupole sont aussi respectivement alourdi et encombrant par la présence de ces multiples éléments optiques.

Les Figures 5 et 6 illustrent les éléments composant le système optique dans un module de lumière et leur agencement possible dans la coupole à éclairage axial selon l'invention.

Sur la Figure 5, on a illustré deux LEDs 10, 20, devant chacune desquelles est positionnée une optique de collimation, ici un collimateur 18, 19, de manière à ce que les faisceaux de lumière émis par les LEDs soient focalisés avant d'atteindre le diviseur de faisceau 13. Un miroir à facette plan 21 est agencé dans la coupole de sorte à pouvoir réfléchir totalement l'un des faisceaux de lumière déjà focalisés et de lumière mélangée résultant vers le champ opératoire.

On a illustré sur la Figure 6 l'agencement d'une coupole à éclairage axial selon un mode de réalisation pour former une tâche d'éclairement 3 à diamètre et température de couleur variables. Dans ce mode de réalisation sont agencés une première couronne de LEDs 10 et une seconde couronne de LEDs 20 coaxiale. Le diviseur de faisceau 13 est un miroir annulaire à facettes semi-réfléchissantes coaxial. Entre chaque couronne de LEDs 10, 20 et le diviseur de faisceau 13, sont agencés des collimateurs 18, 19. Comme encore visible sur cette Figure 6, un miroir annulaire à réflexion totale à facettes planes 21 entoure le miroir annulaire à facettes semi-réfléchissantes 13. Les facettes du miroir annulaire à facettes planes 21 sont sensiblement homothétiques respectivement aux facettes du miroir annulaire à facettes semi-réfléchissantes 13.

Le diviseur de faisceau 13 peut aussi être plusieurs miroirs semi-réfléchissants plans disposés annulairement.

Le miroir à réflexion totale 21 peut être composé de plusieurs miroirs plans d'une facette disposés annulairement.

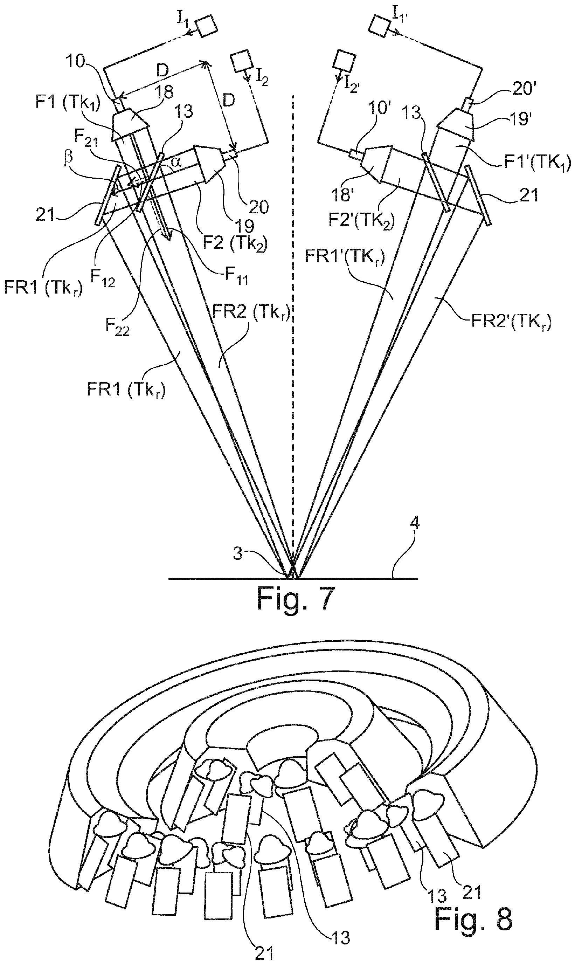

On peut voir maintenant sur la Figure 7, une illustration de la configuration des éléments optiques compris dans deux modules d'éclairage intégrés dans une coupole à éclairage axial AA selon l'invention.

Un premier module de lumière comprend deux LEDs 10, 20. On utilise de préférence des première et seconde LEDs 10, 20 géométriquement identiques présentant des températures de couleur Tk1, Tk2 différentes, provenant du même fournisseur, ayant un même boîtier, des mêmes puces électroniques et nécessitant un même type d'alimentation. Les LEDs 10,20 sont disposées à égales distances du miroir à facettes semi-réfléchissantes 13, la première LED 10 étant tournée de 90° par rapport à la seconde LED 20. Devant chacune des LEDs 10,20 sont agencés respectivement deux optiques de collimation 18, 19 identiques, à égale distance des LEDs 10,20. Ainsi, comme représenté ici, deux faisceaux de lumière collimatés F1 à Tk1 et F2 à Tk2 sont générés. Selon le principe de la division des faisceaux décrit précédemment, les faisceaux F1 et F2 sont divisés par le miroir à facettes semi-réfléchissantes 13, incliné d'un angle α de 45°, pour produire deux faisceaux de lumière mélangée résultants FR1, FR2, ayant une même température de couleur intermédiaire Tkr comprise entre Tk1 et Tk2. Selon l'invention les faisceaux de lumière mélangée résultants FR1 et FR2 sont collimatés. L'optique de collimation peut être une lentille de collimation, un collimateur ou un réflecteur hydride ou parmi tout autre composant remplissant une fonction de collimation intensif. Ainsi, selon l'invention, aucune optique n'est nécessaire pour envoyer sur le champ opératoire 4 le faisceau de lumière mélangée résultant FR2 collimaté pour former la tache d'éclairement 3. Selon l'invention, seul un miroir à facettes planes est agencé pour dévier le faisceau de lumière mélangée résultant FR1 dans le champ opératoire, de manière à ce qu'il se superpose au faisceau de lumière mélangée résultant FR2 pour former ensemble la tache d'éclairement 3. Pour cela le miroir à facettes planes est incliné d'un angle β, compris entre 37 et 57° de sorte à être à réflexion totale.

Sur la figure 7 est représenté un second module de lumière comprenant deux sources de lumière à LEDs 10', 20' identiques, couplées à deux optiques de collimation 18', 19' identiques, générant deux faisceaux de lumière collimatée respectivement F1' de température de couleur Tk1 et F2' de température de couleur Tk2, différente de Tk1. Les faisceaux F1' et F2' sont divisés par le miroir à facettes semi-réfléchissantes 13, incliné d'un angle α de 45°, pour produire deux faisceaux de lumière mélangée résultants FR1', FR2', collimatés ayant une même température de couleur intermédiaire Tkr, identique à la température de couleur intermédiaire du premier module et comprise entre Tk1 et Tk2. Le module est agencé de sorte que le faisceau de lumière mélangée résultant FR2' collimaté se superpose la tache d'éclairement 3. Le faisceau de lumière mélangée résultant FR2' est dévié par le miroir à facettes planes incliné avec un angle β entre 37 et 57°de manière à ce qu'il se superpose au faisceau de lumière mélangée résultant FR1', FR1 et FR2 pour former ensemble la tache d'éclairement 3.

On comprend d'abord que selon l'invention, dans la coupole à éclairage axial, l'intégrité de la fonction de collimation se fait au plus près des LEDs du fait de la présence des optiques de collimation 18, 19. Ainsi par la génération de faisceaux de lumière collimatés, qui sont des faisceaux moins divergents que dans le système décrit dans l'art antérieur, il est possible d'utiliser un diviseur de faisceau à miroir semi-réfléchissant de dimension plus petite que dans l'art antérieur.

De plus, le premier faisceau de lumière mélangé résultant FR1 en sortie du diviseur de faisceaux étant collimaté, il est avantageux de pouvoir remplacer le réflecteur elliptique du système décrit dans l'art antérieur par un miroir plan 21 pour réfléchir ce faisceau de lumière mélangée dans le champ opératoire.

Selon l'invention, la coupole à éclairage axial pourra être moins encombrante en diminuant la distance entre les LEDs et le miroir annulaire à facettes semi-réfléchissantes, mais aussi en optimisant la distance entre le miroir annulaire à facettes semi-réfléchissantes et le miroir annulaire à facettes planes.

Selon l'invention, par rapport à l'art antérieur, le système optique ne comprend plus de lentille. Ainsi, chaque module de lumière comprend un nombre d'éléments réduits. Ainsi, la coupole à éclairage axial selon l'invention est plus légère. De plus, avec cet agencement son encombrement peut être réduit de 40 %, son épaisseur pouvant passer de 12 cm à 7 cm pour une même fonction optique. La coupole d'éclairage est donc plus maniable, améliorant ainsi le confort de l'utilisateur.

Selon l'invention, dans une coupole 9, il y a plusieurs modules de lumière M répartis autour de l'axe d'éclairement AA de sorte que par exemple les LEDs 10 des modules de lumière M forment une première couronne autour de l'axe d'éclairement AA et les LEDs 20 des modules de lumière M forment une seconde couronne autour de l'axe AA coaxiale à la première couronne de LEDs 10. La première couronne de LEDs 10 a par exemple un diamètre supérieur à celui de la seconde couronne de LEDs 20. On a donc dans la coupole 9 une série de diviseurs de faisceau 13 répartis autour de l'axe d'éclairement AA et qui forment un miroir annulaire à facettes semi-réfléchissantes coaxial aux couronnes de LEDs 10,20. Un miroir annulaire à facettes planes entoure le miroir annulaire à facettes semi-réfléchissantes.

Selon l'invention, on utilise le principe de construction du module de lumière M exposé ci-dessus pour réaliser un dispositif d'éclairage 1 notamment en forme de coupole avec lequel on peut former en plus une tache d'éclairement 3 de dimension variable dans le plan de travail qui correspond au champ opératoire 4 distant d'environ 1m de la coupole.

Dans le mode de réalisation de la Figure 6 sont représentés de manière alternée dans des modules de lumières du type M1, M2 avec des sources de lumière à LED identiques, par exemple un premier module M1 comprenant des premières optiques de collimation 18, 19 identiques et qui produisent une tache de lumière de « petite dimension » (module dit petite tache) et par exemple un second module M2 comprenant des secondes optiques de collimation 18', 19' identiques et qui produisent une tache de lumière de « grande dimension » (module dit grande tache). Les deux modules sont agencés de manière à ce que les taches d'éclairement se superposent.. Le nombre de module M1 dit petite tache n'est pas forcément le même que le nombre de module M2 dit grande tache. De par cette configuration, on peut faire varier de diamètre de la tache d'éclairement sans mouvement mécanique, mais en faisant varier le courant d'alimentation des LEDs dans les deux modules, tout en gardant un éclairement central constant.

Pour cela, au moyen de l'UC 41, on pilote l'alimentation électrique 40 de sorte à envoyer sélectivement des courants I1, I2 respectivement dans les LEDs 10, 20 des modules de lumière M1 et des courants I1' et I2' respectivement dans les LEDs 10, 20 des modules de lumière M2 pour modifier la dimension de la tache d'éclairement et/ou modifier la température de couleur comme exposé ci-dessus.

Il faut comprendre que dans cet agencement, pour garder l'homogénéité de température de couleur dans la tache d'éclairement 3 à dimension variable, chaque module de lumière M1 et M2 doit émettre la même température de couleur. En d'autres termes, le ratio I1/I2 doit être identique au ratio I1'/I2' pour obtenir une tache d'éclairement 3 à la température de couleur intermédiaire Tkr et on fait varier ce ratio pour faire varier la température de couleur dans la tache d'éclairement entre Tk1 et Tk2. Pour faire varier le diamètre de la tache d'éclairement, on fait varier le courant relatif entre les modules de lumière M1 et M2, c'est à dire le ratio I1/I1' et donc I2/I2'.

Pour une température de couleur intermédiaire Tkr fixe entre Tk1 et Tk2, on peut faire varier I1' et I1 ou I2' et I2 selon le principe des vases communicants pour faire varier le diamètre de la tache d'éclairement entre un grand diamètre (le diamètre de la tache produite par les modules de lumière M2 seuls) et un petit diamètre (le diamètre de la tache produite par les modules de lumière M1 seuls). A noter qu'il faut que la somme des courants I1+I1' ou I2+I2' soit sensiblement constante pour avoir une variation de diamètre de tache à éclairement constant, tout en conservant le ratio I1/I2 égal à I1'/I2' pour conserver la même température de couleur dans la tache d'éclairement 3.

Plus particulièrement, si les courants I1' et I2' sont nuls, la tâche d'éclairement 3 est produite que par les modules de lumière M1 seuls et on a donc une petite tache d'éclairement avec un diamètre de par exemple 10cm.

Si les courants I1 et I2 sont nuls, la tache d'éclairement 3 est produite que par les modules de lumière M2 seuls et on a donc une grande tache d'éclairement avec un diamètre de par exemple 20 cm.

Si on fournit des courants non nuls I1 et I2 aux modules de lumière M1 et des courants non nuls I1' et I2' aux modules de lumière M2, on produit une tache d'éclairement avec un diamètre intermédiaire entre 10 et 20 cm.

Pour garder un même niveau d'éclairement pour les différents diamètres de tache d'éclairement, l'UC 41 ajuste le courant dans toutes les LEDs des modules de lumière M1 et M2 tout en gardant un ratio I1'/I2' identique au ratio I1/I2 de façon à ne pas changer la température de couleur pour la tache d'éclairement 3. Si on passe d'une tache d'éclairement d'un certain diamètre à une tache d'éclairement plus grande, l'UC 41 doit augmenter les courants avec la même proportion pour chaque LED.

Dans un autre mode de réalisation illustré sur la Figure 8, dans la coupole 9, les modules M peuvent forment deux couronnes de modules coaxiales entre elles suivant l'axe d'éclairage central AA.

Le dispositif d'éclairage selon l'invention opératoire comprend une coupole à éclairage axial présentant un encombrement et un nombre de pièces réduits, avec des sources de lumière, un système optique et une alimentation en courants des sources de lumière qui sont agencés pour placer les faisceaux lumineux issus des sources selon différentes configurations de superposition des faisceaux lumineux dans le plan de superposition qui correspond au plan de travail où est formée la tache d'éclairement, ces différentes configurations correspondant à différentes dimensions ou différents diamètres de la tache d'éclairement.

## Revendications

1. Dispositif d'éclairage (1) pour l'éclairage d'un champ opératoire (4) comprenant dans une coupole (9) à éclairage axial une première couronne de LEDs (10) apte à émettre un premier faisceau (F1) de lumière à une première température de couleur (Tk1) et une seconde couronne de LEDs (20), coaxiale à ladite première couronne de LEDs et apte à émettre un second faisceau (F2) de lumière à une seconde température de couleur (Tk2) différente de ladite première température de couleur et un miroir annulaire à facettes semi-réfléchissantes (13) coaxial pour mélanger lesdits faisceaux de lumière émis (F1, F2) par lesdites deux couronnes de LEDs (10, 20) et former deux faisceaux de lumière mélangée résultants (FR1, FR2) ayant une même température de couleur intermédiaire (Tkr) comprise entre ladite première température de couleur (Tk1) et ladite seconde température de couleur (Tk2), et un système optique annulaire qui entoure ledit miroir annulaire à facettes semi-réfléchissantes (13) pour réfléchir ledit premier faisceau de lumière mélangée résultant (FR1) dans ledit champ opératoire (4) pour le combiner avec ledit second faisceau de lumière mélangée résultant (FR2) suivant une certaine configuration de superposition dans un plan de superposition où est formée une tache d'éclairement (3) à ladite température de couleur intermédiaire (Tkr), dans lequel il est prévu des optiques de collimation (18, 19) entre chaque couronne de LEDs (10, 20) et ledit miroir annulaire à facettes semi-réfléchissante (13), **caractérisé en ce que** ledit système optique annulaire qui entoure ledit miroir annulaire à facettes semi-réfléchissantes (13) est un miroir annulaire à réflexion totale à facettes planes (21) sensiblement homothétiques respectivement auxdites facettes semi-réfléchissantes.

2. Dispositif d'éclairage selon la revendication 1 **caractérisée en ce que** lesdites optiques de collimation (18, 19) sont des lentilles.

3. Dispositif d'éclairage selon la revendication 1 **caractérisée en ce que** lesdites optiques de collimation (18, 19) sont des collimateurs.

4. Dispositif d'éclairage selon la revendication 1 **caractérisée en ce que** lesdites optiques de collimation (18, 19) sont des réflecteurs hybride.

5. Dispositif d'éclairage selon la revendication 1 **caractérisée en ce qu'**une alimentation électrique (40) est prévue pour alimenter en courants lesdites LEDs (10, 20) et moduler lesdits courants (11,12).

6. Dispositif d'éclairage selon la revendication 1 **caractérisée en ce que** dans ladite première couronne de LEDs, les LEDs (10) sont couplées de manière alternée à une première (18) et un seconde (18') optique de collimation, ladite seconde optique de collimation (18') étant différente de ladite première optique de collimation (18) et dans ladite seconde couronne de LEDs les LEDs 20 sont couplées de manière alternée à une troisième (19) et un quatrième (19') optique de collimation, lesdites première (18) et troisième (19) optiques de collimation étant identiques et lesdites seconde (18') et quatrième (19') optiques de collimation étant identiques, pour former respectivement une première tache d'éclairement et une seconde tache d'éclairement de diamètres différents.

7. Dispositif d'éclairage selon les revendications 5 et 6, **caractérisé en ce que** l'alimentation électrique (40) pour alimenter en courants (I1,I2,I1',I2') lesdites LEDs (10,20) et moduler lesdits courants permet de modifier la dimension de la tache d'éclairement (3) et/ou de modifier la température de couleur (Tkr) de la tache d'éclairement (3).

8. Dispositif d'éclairage selon la revendication 5 **caractérisée en ce que** l'alimentation électrique (40) est agencée en outre pour moduler lesdits courants de telle façon à former une tache d'éclairement à un niveau d'éclairement constant.

9. Dispositif d'éclairage selon les revendications précédentes, **caractérisé en ce que** ledit miroir annulaire à facettes semi-réfléchissantes (13) comprend plusieurs miroirs semi-réfléchissants plans disposés annulairement.

10. Dispositif d'éclairage selon les revendications précédentes, **caractérisé en ce que** ledit miroir annulaire à réflexion totale à facettes planes (13) comprend plusieurs miroirs plans d'une facette disposés annulairement.

## Patentansprüche

1. Beleuchtungsvorrichtung (1) zur Beleuchtung eines Operationsfeldes (4), umfassend in einer Kuppel (9) zur axialen Beleuchtung einen ersten LED-Kranz (10), der geeignet ist, einen ersten Lichtstrahl (F1) mit einer ersten Farbtemperatur (Tk1) zu entsenden, und einen zweien LED-Kranz (20) koaxial zum ersten LED-Kranz und geeignet, einen zweiten Lichtstrahl (F2) mit einer zweiten Farbtemperatur (Tk2), die sich von der ersten Farbtemperatur unterscheidet, zu entsenden, und einen koaxialen ringförmigen Spiegel mit halb-reflektierenden Facetten (13), um die von den zwei LED-Kränzen (10, 20) entsandten Lichtstrahlen (F1, F2) zu mischen und zwei resultierende Mischlichtstrahlen (FR1, FR2) zu bilden, die eine selbe Zwischenfarbtemperatur (Tkr) zwischen der ersten Farbtemperatur (Tk1) und der zweiten Farbtemperatur (Tk2) haben, und ein ringförmiges optisches System, das den ringförmigen Spiegel mit halb-reflektierenden Facetten (13) umgibt, um den ersten resultierenden Mischlichtstrahl (FR1) in das Operationsfeld (4) zu reflektieren, um ihn mit dem zweiten resultierenden Mischlichtstrahl (FR2) nach einer gewissen Überlagerungsausführung in einer Überlagerungsebene zu kombinieren, in der ein Leuchtfleck (3) mit der Zwischenfarbtemperatur (Tkr) gebildet ist, wobei Kollimationsoptiken (18, 19) zwischen jedem LED-Kranz (10, 20) und dem ringförmigen Spiegel mit halb-reflektierenden Facetten (13) vorgesehen sind,
**dadurch gekennzeichnet, dass** das ringförmige optische System, das den ringförmigen Spiegel mit halb-reflektierenden Facetten (13) umgibt, ein ringförmiger Spiegel mit Totalreflexion mit ebenen Facetten (21) ist, die im Wesentlichen zu den halb-reflektierenden Facetten homothetisch sind.

2. Beleuchtungsvorrichtung nach Anspruch 1, **dadurch gekennzeichnet**, das die Kollimationsoptiken (18, 19) Linsen sind.

3. Beleuchtungsvorrichtung nach Anspruch 1, **dadurch gekennzeichnet, dass** die Kollimationsoptiken (18, 19) Kollimatoren sind.

4. Beleuchtungsvorrichtung nach Anspruch 1, **dadurch gekennzeichnet, dass** die Kollimationsoptiken (18, 19) Hybridreflektoren sind.

5. Beleuchtungsvorrichtung nach Anspruch 1, **dadurch gekennzeichnet, dass** eine elektrische Versorgung (40) vorgesehen ist, um die LEDs (10, 20) mit Strom zu versorgen und die Ströme (I1, I2) zu modulieren.

6. Beleuchtungsvorrichtung nach Anspruch 1, **dadurch gekennzeichnet, dass** in dem ersten LED-Kranz die LEDs (10) alternierend mit einer ersten (18) und einer zweiten (18') Kollimationsoptik gekoppelt sind, wobei die zweite Kollimationsoptik (18') zu der ersten Kollimationsoptik (18) unterschiedlich ist, und dass in dem zweiten LED-Kranz die LEDs (20) alternierend mit einer dritten (19) und einer vierten (19') Kollimationsoptik gekoppelt sind, wobei die erste (18) und die dritte (19) Kollimationsoptik identisch sind, und die zweite (18') und die vierte (19') Kollimationsoptik identisch sind, um einen ersten Leuchtfleck bzw. einen zweiten Leuchtfleck mit unterschiedlichen Durchmessern zu bilden.

7. Beleuchtungsvorrichtung nach den Ansprüchen 5 und 6, **dadurch gekennzeichnet, dass** es die elektrische Versorgung (40), um die LEDs (10, 20) mit Strom (I1, I2, I1', I2') zu versorgen, und die Ströme zu modulieren, ermöglicht, die Dimension des Leuchtflecks (3) zu verändern und/oder die Farbtemperatur (Tkr) des Leuchtflecks (3) zu verändern.

8. Beleuchtungsvorrichtung nach Anspruch 5, **dadurch gekennzeichnet, dass** die elektrische Versorgung (40) ferner eingerichtet ist, um die Ströme zu modulieren, um einen Leuchtfleck auf einem konstanten Leuchtniveau zu bilden.

9. Beleuchtungsvorrichtung nach den vorhergehenden Ansprüchen, **dadurch gekennzeichnet, dass** der ringförmige Spiegel mit halb-reflektierenden Facetten (13) mehrere ringförmig angeordnete ebene halb-reflektierende Spiegel umfasst.

10. Beleuchtungsvorrichtung nach den vorhergehenden Ansprüchen, **dadurch gekennzeichnet, dass** der ringförmige Spiegel mit Totalreflexion mit ebenen Facetten (13) mehrere ringförmig angeordnete ebene Spiegel einer Facette umfasst.

## Claims

1. A lighting device (1) for illuminating an operative field (4), the lighting device comprising, in an axial lighting dome (9), a first ring of LEDs (10) suitable for emitting a first light beam (F1) at a first color temperature (Tk1), and a second ring of LEDs (20) about the same axis as said first ring of LEDs and suitable for emitting a second light beam (F2) at a second color temperature (Tk2) different from said first color temperature, and an annular facetted mirror having semi-reflective facets (13) and arranged about said axis for mixing said light beams (F1, F2) emitted by said two rings of LEDs (10, 20) and for forming two resulting mixed light beams (FR1, FR2) having the same intermediate color temperature (Tkr) lying between said first color temperature (Tk1) and said second color temperature (Tk2), and an annular optical system that surrounds said annular facetted mirror having semi-reflective facets (13) so as to reflect said first resulting mixed light beam (FR1) onto said operative field (4) to combine it with said second resulting mixed light beam (FR2) in a certain superposition configuration in a superposition plane in which an illumination spot (3) is formed at said intermediate color temperature (Tkr), wherein collimation optical systems (18, 19) are provided between each ring of LEDs (10, 20) and said annular facetted mirror having semi-reflective facets (13), said lighting device being **characterized in that** said annular optical system that surrounds said annular facetted mirror having semi-reflective facets (13) is a total reflection annular facetted mirror having plane facets (21) that are substantially geometrically similar to said semi-reflective facets.

2. A lighting device according to claim 1, **characterized in that** said collimation optical systems (18, 19) are lenses.

3. A lighting device according to claim 1, **characterized in that** said collimation optical systems (18, 19) are collimators.

4. A lighting device according to claim 1, **characterized in that** said collimation optical systems (18, 19) are hybrid reflectors.

5. A lighting device according to claim 1, **characterized in that** an electrical power supply (40) is provided for feeding currents to said LEDs (10, 20) and for modulating said currents (II, 12).

6. A lighting device according to claim 1, **characterized in that**, in said first ring of LEDs, the LEDs (10) are coupled in alternating manner to a first collimation optical system (18) and to a second collimation optical system (18'), said second collimation optical system (18') being different from said first collimation optical system (18), and, in said second ring of LEDs, the LEDs (20) are coupled in alternating manner to a third collimation optical system (19) and to a fourth collimation optical system (19'), said first and third collimation optical systems (18, 19) being identical, and said second and fourth collimation optical systems (18', 19') being identical, so that they form respectively a first illumination spot and a second illumination spot, the two spots being of different diameters.

7. A lighting device according to claims 5 and 6, **characterized in that** the electrical power supply (40) for feeding currents (II, I2, I1', I2') to said LEDs (10, 20), and for modulating said currents makes it possible to modify the size of the illumination spot (3) and/or to modify the color temperature (Tkr) of the illumination spot (3).

8. A lighting device according to claim 5, **characterized in that** the electrical power supply (40) is also arranged to modulate said currents in such a manner as to form an illumination spot at a constant illumination level.

9. A lighting device according to any preceding claim, **characterized in that** said annular facetted mirror having semi-reflective facets (13) comprises a plurality of plane semi-reflective mirrors disposed annularly.

10. A lighting device according to any preceding claim, **characterized in that** said total-reflection annular facetted mirror having plane facets (13) comprises a plurality of plane one-facet mirrors disposed annularly
